# EUROPEAN PATENT APPLICATION

(11) **EP 2 772 795 A1**
(43) Date of publication of application: **03.09.2014**
(21) Application number: 13157188.7
(22) Date of filing: 28.02.2013
(51) Int. Cl.: G02C 13/00, G03B 35/10, G02B 5/04, A61B 3/11, G02C 11/00

(54) **Method, system and device for improving optical measurement of ophthalmic spectacles**

(71) Applicant: Shamir Optical Industry Ltd, 12135 Upper Galilee (IL); Codi Comercio Design Industrial, Lda, 2415-456 Leiria (PT)
(72) Inventor: Dias Da Silva, Luis Filipe Feijo, 4450-256 Matosinhos (PT); Domingues, Fernando Moises Fernandes, 2430-017 Marinha Grande (PT)
(74) Representative: Pearl Cohen Zedek Latzer Baratz UK LLP

(57) **Abstract**

A system and method for optical measurement, the system comprising a camera having a lens unit and an image sensor, the image sensor configured to generate image data based on light received onto the sensor; a prism device to receive light from two different angles and to relay the light through the lens unit onto respective two portions of the image sensor; and a processor to receive image data from the two portions of the image sensor; identify key points of the image data from the two portions; create a three-dimensional model of an object based on the image data from the two portions; and calculate based on the key points parameters of the object.

## Description

### FIELD OF THE INVENTION

This invention relates to measurements of ophthalmic spectacles, and in particular to improving of the measurement methods for eyeglass frame parameters and optical data for individual wearer.

### BACKGROUND OF THE INVENTION

A common solution for people suffering from poor visual acuity is the use of corrective lenses. These may come in the form of corrective contact lenses or corrective lenses fitted into spectacles. When spectacles are used, the spectacle frame may have one of many different sizes and/or shapes. The size and the shape of the spectacle frame affects the positioning of the optical lenses relative to the eyes of the user, and therefore affects, among other things, the fitting height, the back vertex distance (BVD, distance between the back side of the lens and the eye), nasal-pupil distance (that in known methods is measured relative to the middle of the nose) and the panoramic and pantoscopic tilts (horizontal and vertical angles, respectively) of the lens.

In recent years many of the ophthalmic lens manufacturers introduced state of the art optimization algorithms to compensate the difference between the optometrist measurements of the lens optical characteristics in the examination room and the lens optical performance as perceived by the patient. These algorithms can be used to design single vision, bi-focal, and progressive lenses and usually compensate for differences in the oblique refraction and induced cylinder power caused by the frame pantoscopic and panoramic tilts and by the back vertex distance (BVD) difference between the positioning of the trial frame or phoropter during the refraction examination and the positioning of the fitted spectacle frame.

The panoramic tilt is commonly measured off the spectacle frame, with the assumption that when the frame is fitted on the patient's face, the angle will not change. This assumption might results in significant inaccuracies of this measurement, due to the patient facial structure. Measuring of the pantoscopic tilt on the patient's face requires a more complicated procedure. Sometimes the pantoscopic tilt is completely disregarded. The BVD can be measured using a distometer. However, the measurements are inaccurate, uncomfortable for the patient and cumbersome for the clinician, and therefore usually a default BVD value is used instead.

Recent attempts have been made to measure the abovementioned parameters taking images of the patient wearing a selected frame. However, those familiar with the art are aware that such systems require a calibration in order to obtain a directional reference, i.e. to be able to determine the position and rotation of the head in the image. In order to do this, such systems usually require a fixture with a known pattern to be fastened to the frames during the measurement, such as a structure clipped on the frame as reference. Some of these systems are dedicated standalone systems, and are quite expensive. These systems include cameras, light sources, dedicated computers, and extensive software, while other can work on tablets, but since the 3D abilities of all of these systems are very limited, they must take at least two images of the patient from the front, and side positions.

### SUMMARY OF THE INVENTION

Embodiments of the present invention may provide a system for optical measurement, the system may include: a camera having a lens unit and an image sensor, the image sensor configured to generate image data based on light received onto the sensor; a prism device to receive light from at least two different angles and to relay the light through the lens unit onto respective at least two portions of the image sensor; and a processor to: receive image data from the at least two portions of the image sensor; identify key points of the image data from the at least two portions; create a three-dimensional model of an object based on the image data from the at least two portions; and calculate based on the key points parameters of the object.

Additionally, embodiments of the present invention may provide a method for optical measurement, the method may include: receiving image data from at least two portions of an image sensor of a camera having a lens unit and an image sensor, the image sensor configured to generate image data based on light received onto the sensor, wherein the light is received by a prism device from at least two different angles and relayed through the lens unit onto respective at least two portions of the image sensor; identifying key points of the image data from the at least two portions; creating a three-dimensional model of an object based on the image data from the at least two portions; and calculating based on the key points parameters of the object.

According to embodiments of the present invention, the processor may receive indications by a user of the key points, and/or the processor may identify the key points automatically. In some embodiments, the processor may identify at least some of the key points by pupil detection methods.

According to embodiments of the present invention, the processor may calculate parameters that are pre-determined or requested by a user.

According to embodiments of the present invention, the camera and processor may be included in a portable computer device. The computer device may include a display to display at least two generated images from the respective at least two portions of the image sensor, and the processor may receive indications by a user of the key points, calculate three-dimensional locations of the key points and/or calculate the parameters of the object based on these locations.

According to some embodiments of the present invention, the prism device may be installed onto the lens unit.

According to some embodiments of the present invention, the object may include a spectacles frame on a patient's face and the calculated parameters may include at least one of a list including pupil distances, frame size, distance between lenses, fitting heights, panoramic tilt, pantoscopic tilt and back vertex distance. The identified key points may include at least one of a list including: centers of the right and left pupils, horizontal and vertical extremes of the lenses when the lenses are held in a spectacles frame, and the lens extremes along the vertical line passing through a center of a pupil, when the respective lens is held upright in a spectacles frame.

According to some embodiments of the present invention, the prism device may include a housing, tubes through which light may enter the prism device, side mirrors and a central prism unit onto which light may be reflected by the side mirrors, wherein the central prism unit may reflect the light from two of the tubes onto two respective portions of the image sensor via the lens unit.

According to some embodiments of the present invention, the processor may calibrate the position of the prism device relative to the object before the image data is captured.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

Figs. 1A and 1B are schematic illustrations of a system for improving optical measurement of ophthalmic spectacles according to embodiments of the present invention;

Fig. 2 is a schematic illustration of an image captured by the system according to embodiments of the present invention;

Fig. 3 is a schematic illustration of the output parameters calculated by a processor according to embodiments of the present invention; and

Fig. 4 is a schematic flow chart illustrating a method improving optical measurement of ophthalmic spectacles according to embodiments of the present invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

Embodiments of the present invention provide method and system and device for improving optical measurement of ophthalmic spectacles. The method and system according to embodiments of the present invention may utilize a portable optical device that can be attached to a portable computer device such as, for example, a tablet computer, for example along with a compatible program that may run on the portable computer device. According to embodiments of the present invention, all the required optical measurements may be performed based on a single camera operation. By using the attached optical device, one can take, according to embodiments of the present invention, a split up photo of a patient's face wearing ophthalmic spectacles from at least two different angles, by a single camera operation, i.e. a single shot, without having any equipment installed on the spectacles frame or on the patient's face.

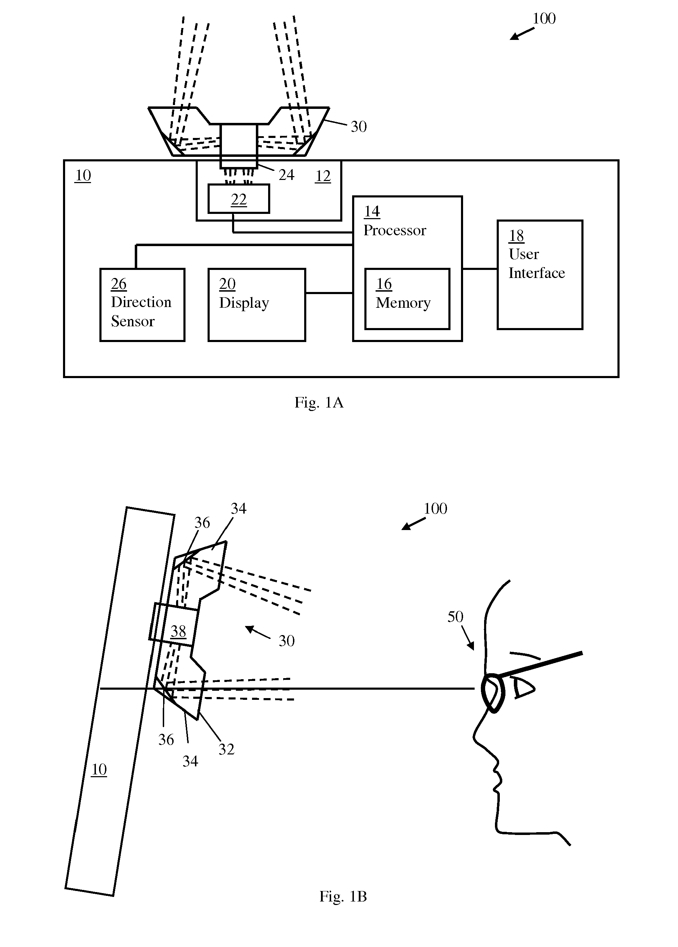

Reference is now made to Figs. 1A and 1B, which are schematic illustrations of a system 100 for improving optical measurement of ophthalmic spectacles according to embodiments of the present invention. System 100 may include a portable computer device 10 and a portable optical prism device 30. Computer device 10 may be, for example, a hand-held computer, a smartphone, a tablet computer or any other suitable device. Computer device 10 may include a camera 12 that may include, for example, an image sensor 22 and a lens unit 24. Image sensor 22 may receive light through lens unit 24 and may generate image data based on the received light. Additionally, computer device 10 may include a processor 14, a memory 16, a user interface 18, a display 20 and a direction sensor 26. Processor 14 may execute software or instructions (e.g. stored in memory 16) to carry out methods as disclosed herein. Memory 16 may include an article such as a computer or processor readable non-transitory storage medium, such as for example a memory card, a disk drive, or a USB flash memory encoding, including or storing instructions, e.g., computer-executable instructions. When executed by a processor or controller such as processor 14, the instructions stored and/or included in memory 16 may cause the processor or controller to carry out methods disclosed herein. User interface 18 may be, for example, executed by, or may be part of, processor 14.

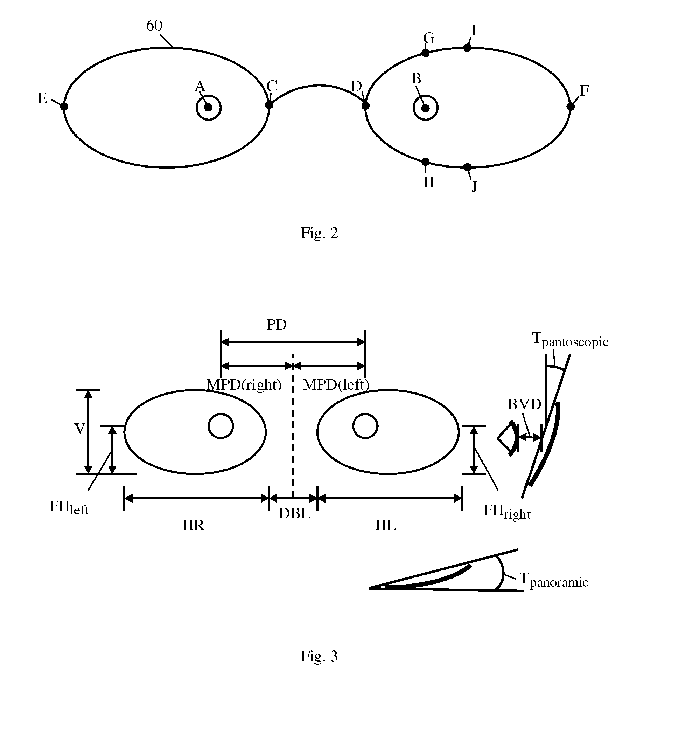

Optical prism device 30 may be installed onto lens unit 24 of camera 12. Prism device 30 may receive light from at least two different angles and may relay the received light through lens unit 24 onto respective at least two portions of image sensor 22. Therefore, two sets of image data that may generate two images from two different angles of an object 50 such as, for example, a spectacles frame, may be obtained from the two respective portions of image sensor 22. The image data that may constitute the two images may be captured by a single camera operation, such as, for example by a single click/tap on a camera button. Processor 14 may receive the image data from the two portions of image sensor 22 and may identify key points of said image data (shown in Fig. 2), e.g. couples of key points in the two sets of image data, each couple may represent the same point on the actual object 50. Based on the image data from said two portions, processor 14 may create a stereoscopic image, e.g. a three-dimensional model of an object 50 such as, for example, a spectacles frame on a patient's face. For example, processor 14 may create the stereoscopic image by matching the indicated key point couples or other pairs of points on the two images identified by processor 14 as representing the same point of the actual object 50, and by calculating distances of each point by triangulation methods or by any other known three-dimensional modeling method. Additionally, processor 14 may identify/calculate a three-dimensional location of each of said key points, for example relative to absolute coordinates of computer device 10. Based on the identified key points and/or the identified three-dimensional locations of the key points, processor 14 may calculate certain parameters of the object 50, as described in detail below with reference to Figs. 2 and 3. As described in detail herein below, the calculated parameters of a spectacles frame on a patient's face may include, for example, pupil distances (PD) such as left and right mono pupil distances, frame size, distance between lenses (DBL), fitting heights, panoramic tilt (horizontal tilt of the lens in the frame), pantoscopic tilt (vertical tilt of the lens in the frame) and back vertex distance (BVD, distance between the back side of the lens and the eye). The calculated parameters can be saved and/or, for example, emailed in a formatted file, and/or can be sent to an online ordering system. The calculated parameters may be pre-determined parameters in instructions stored in memory 16 and/or parameters requested by a user.

In some embodiments of the present invention, the two generated images from the respective two portions of the image sensor may be displayed by display 20. A user may identify the key points and indicate the key points, for example by user interface 18, on each of the two images or on a created three-dimensional image by processor 14. Based on the identified key points, processor 14 may identify three-dimensional locations of the key points and/or calculate certain parameters of the object 50 based on these locations.

Prism device 30 may include a housing 32, tubes 34, side mirrors 36 and central prism unit 38. Light may enter prism device 30 via tubes 34 and may be reflected by side mirrors 36 onto central prism unit 38, which may reflect the light from the two tubes 34 onto two respective portions of image sensor 22 via lens unit 24, as described herein.

Before the image data is captured, processor 14 may calibrate the position of device 30 relative to object 50. for example, in order to obtain a frontal photo of a patient's face wearing a spectacles frame or other object 50, the tablet may be held at a small inclination toward the patient/object 50, for example of about 10 degrees. The prism inclination angle relative to the tablet (or other computer device) 10, when device 30 is installed on the tablet, may be, for example, of about 45-55 degrees. In order to capture a stereoscopic image of the patient's face or other object 50, the distance between device 10 and the patient may be, for example, about 45-50 centimeters. The lower tube 34 may be substantially aligned horizontally with the patient's eyes or other face portion or other object 50 portion as may be calibrated and/or adjusted, or a horizontal line may otherwise determined, before the image is captured. Based on the determined horizontal line, the prism angles may be calculated relative to the horizontal line. The horizontal distance between the side mirrors 36 can be calculated and/or adjusted before the image is captured. Direction sensor 26 may determine the absolute world vertical line and may facilitate calibrations of absolute directions such as the horizontal line.

Reference is now made to Fig. 2, which is a schematic illustration of an image captured by system 100 according to embodiments of the present invention. Fig. 2 illustrates one of the two images captured on the two respective portions of image sensor 22. For each of the two images, processor 14 and/or a user may identify a set of key points. The key points may be identified automatically by processor 14, for example, by image processing methods, or the image may be displayed by display 20 and a user may mark the key points by user interface 18, for example by tapping a touch-screen or using other input methods of device 10. The identified key points may include A and B, which are centers of the right and left pupils, respectively, E, C, D, I, J and F, which are horizontal and vertical extremes of the lenses when the lenses are held in a spectacles frame, and G and H, which are the lens extremes along the vertical line passing through B, when the respective lens is held upright in a spectacles frame. In some embodiments of the present invention, the center of pupil points A and B and/or other key points may be detected by infra-red illumination or other pupil detection method.

After calculating a three-dimensional location of each key point based on the three dimensional model of the lens, processor 14 may calculate the parameters of the spectacles frame on the patient's face as mentioned herein.

Reference is now made to Fig. 3, which is a schematic illustration of the output parameters calculated by processor 14 according to embodiments of the present invention. The output parameters may be calculated based on the indicated or identified key-points and based on a three-dimensional model of the spectacles frame worn on the patient's face. As discussed above, the three-dimensional model may be created, for example, based the two images taken by the two respective portions of image sensor 22. As mentioned above, the three-dimensional model may be created by triangulation or by any other known three-dimensional modeling method.

According to some embodiments of the present invention, the parameters of a spectacles frame on a patient's face that may be calculated by processor 14 may include, for example, mono pupil distance (MPD) and pupil distance (PD), i.e. the distance between pupils, vertical and left horizontal and right horizontal frame maximum sizes (V and HL and HR, respectively), distance between lenses (DBL), left and right fitting heights (FHleft and FHright), i.e. the absolute height from the lens' bottom to the pupil's level, panoramic tilt (Tpanoramic), i.e. horizontal tilt of the lens in the frame, Pantoscopic tilt (Tpantoscopic), i.e. vertical tilt of the lens in the frame and back vertex distance (BVD), i.e. distance between the back side of the lens and the eye.

Processor 14 may calculate some parameters based on the identified key points indicated in Fig. 2. For example, processor 14 may use vector calculus and geometry methods jointly with direction calibration facilitated by direction sensor 26. As mentioned above, in some embodiments of the present invention, some key points such as, for example the center of pupil points A and B may be identified by pupil detection methods such as, for example, methods including infra-red illumination. Therefore, the detection of some of the key points and/or the calculation of some of the parameters such as, for example, the PD, BVD, pantoscopic and panoramic tilts and other parameters may be performed by using infra-red illumination pupil detection methods or other pupil detection methods.

For example, processor 14 may calculate a vector h, equal to E-F. Then, processor 14 may calculate the projection A(J,h), i.e. the projection of point A into the line having the direction h and including the point J. Then, processor 14 may calculate the right fitting height FHright by calculating the verticalized vector (A-A(J,h))v, i.e. projecting the vector A-A(J,h) into a line having the world vertical direction, and taking the absolute size of the verticalized vector. Verticalizing means projection of a point or vector into a line including the origin and having the world vertical direction, provided, for example, by direction sensors 26 of device 10. Then, processor 14 may calculate the left fitting height FHleft by verticalizing the vector B-J and taking the absolute size of the verticalized vector. A verticalized point or vector may be represented herein by the superscript v.

Accordingly, for example, processor 14 may calculate the vertical size V by taking the absolute size of the vector Iv-Jv. Additionaly, processor 14 may calculate the horizontal sizes HL and HR by taking the absolute sizes of the vectors D-F and C-E, respectively. The DBL may be calculated by processor 14 by taking the absolute size of vector C-D.

Similarly and/or by additional/other vector calculus and geometry methods processor 14 may calculate other parameters such as, for example, the right and left mono pupil distance (MPD(right) and MPD(left)), the distance between pupils (PD) panoramic tilt (Tpanoramic), Pantoscopic tilt (Tpantoscopic), and back vertex distance (BVD) and/or other parameters. The method and system according to embodiments of the present invention may enable calculation of the MPD(right) and MPD(left) with an error of, for example, ±0.5 millimeters, the fitting heights with an error of, for example, ±1 millimeters, the pantoscopic tilt and panoramic angles with an error of, for example, ±1 degree and the BVD with an error of, for example, ±1 millimeters.

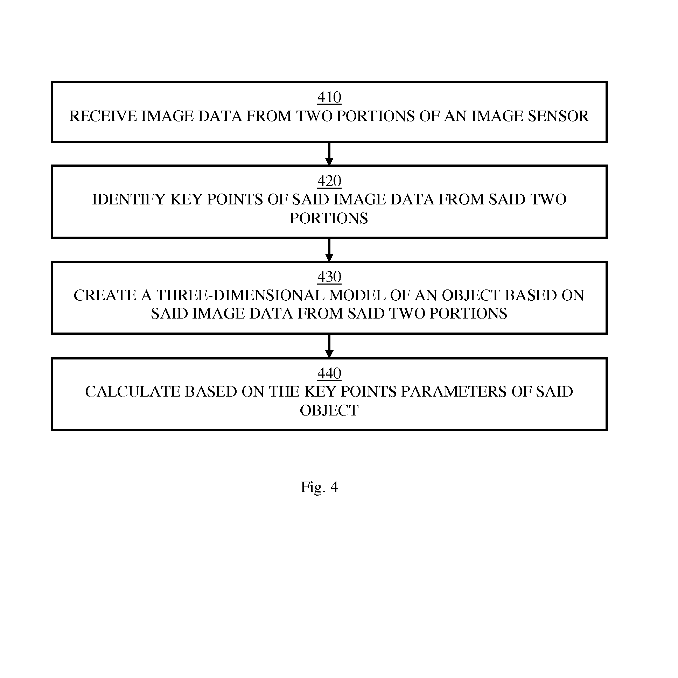

Reference is now made to Fig. 4, which is a schematic flow chart illustrating a method improving optical measurement of ophthalmic spectacles according to embodiments of the present invention. As indicated in block 410, the method may include receiving image data from two portions of an image sensor of a camera having a lens unit and an image sensor, for example as described in detail above. As described, the image sensor may be configured to generate image data based on light received onto the sensor, wherein the light may be received by a prism device from two different angles and may be relayed through said lens unit onto respective two portions of the image sensor. As indicated in block 420, the method may include identifying key points of the image data from said two portions, for example as described in detail above. As indicated in block 430, the method may include creating a three-dimensional model of an object based on the image data from the two portions, for example as described in detail above. As indicated in block 440, the method may include calculating based on the key points parameters of the object, for example as described in detail above.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A system for optical measurement, the system comprising:
a camera having a lens unit and an image sensor, the image sensor configured to generate image data based on light received onto said sensor;
a prism device arranged to receive light from two different angles and to relay said light through said lens unit onto respective two portions of the image sensor; and
a processor configured to:
receive image data from said two portions of the image sensor;
identify key points of said image data from said two portions;
create a three-dimensional model of an object based on said image data from said two portions; and
calculate based on the key points parameters of said object.

2. The system of claim 1, wherein said processor is configured to perform one or more of:
receiving indications by a user of said key points;
identifying the key points automatically; and
identifying at least some of the key points by pupil detection methods

3. The system of claim 1 or claim 2, wherein said processor is configured to calculate parameters that are pre-determined or requested by a user.

4. The system of any preceding claim, wherein said prism device is installed onto said lens unit and comprises a housing, tubes through which light can enter the prism device, side mirrors and a central prism unit onto which light can be reflected by the side mirrors, wherein the central prism unit is arranged to reflect the light from two of said tubes onto two respective portions of the image sensor via said lens unit.

5. The system of any preceding claim, wherein said object is a spectacles frame and wherein the calculated parameters comprise at least one of: pupil distances,
frame size,
distance between lenses,
fitting heights,
panoramic tilt, pantoscopic tilt,
and back vertex distance;
and/or wherein the identified key points comprise at least one of:
centers of the right and left pupils,
horizontal and vertical extremes of the lenses when the lenses are held in a spectacles frame, and
the lens extremes along the vertical line passing through a center of a pupil when the respective lens is held upright in a spectacles frame.

6. The system of any preceding claim, wherein said processor is configured to calibrate the position of the prism device relative to the object before said image data is captured.

7. A computer device comprising a system as claimed in any preceding claim, wherein said computer device comprises a display to display two generated images from the respective two portions of the image sensor, and wherein said processor is to receive indications by a user of the key points, to calculate three-dimensional locations of the key points and to calculate the parameters of the object based on these locations.

8. A method of optical measurement, the method comprising:
receiving image data from two portions of an image sensor of a camera having a lens unit and an image sensor, the image sensor configured to generate image data based on light received onto said sensor, wherein said light is received by a prism device from two different angles and relayed through said lens unit onto respective two portions of the image sensor;
identifying key points of said image data from said two portions;
creating a three-dimensional model of an object based on said image data from said two portions; and
calculating based on the key points parameters of said object.

9. The method of claim 8, comprising one or more of: receiving indications by a user of said key points, identifying the key points automatically, and identifying at least some of the key points by pupil detection methods.

10. The method of claim 8 or claim 9, comprising calculating parameters that are pre-determined or requested by a user.

11. The method of claim 8, 9 or 10, executed by a processor wherein said camera and processor are included in a portable computer device, wherein said computer device comprises a display to display two generated images from the respective two portions of the image sensor, the method comprising receiving indications by a user of the key points, calculating three-dimensional locations of the key points and calculating the parameters of the object based on these locations.

12. The method of any of claims 8 to 11, wherein said prism device is installed onto said lens unit and comprises a housing, tubes through which light can enter the prism device, side mirrors and a central prism unit onto which light can be reflected by the side mirrors, wherein the central prism unit can reflect the light from two of said tubes onto two respective portions of the image sensor via said lens unit.

13. The method of any of claims 8 to 12, wherein said object is a spectacles frame on a patient's face and wherein the calculated parameters comprise at least one of:
pupil distances,
frame size,
distance between lenses,
fitting heights,
panoramic tilt,
pantoscopic tilt and back vertex distance;
and/or wherein the identified key points comprise at least one of:
centers of the right and left pupils,
horizontal and vertical extremes of the lenses when the lenses are held in a spectacles frame, and
the lens extremes along the vertical line passing through a center of a pupil when the respective lens is held upright in a spectacles frame.

14. The method of any of claims 8 to 13, comprising calibrating the position of the prism device relative to the object before said image data is captured.

15. A computer readable medium comprising instructions which when implemented by a processor in a computing system cause the system to implement the method of any of claims 8 to 14.
